# EUROPEAN PATENT APPLICATION

(11) **EP 4 763 084 A1**
(43) Date of publication of application: **24.06.2026**
(21) Application number: 25226459.3
(22) Date of filing: 22.12.2025
(51) Int. Cl.: A61B 5/259, A61B 5/282

(54) **HYDROGEL CONTAINMENT SOLUTIONS FOR THIN-ADHESIVE BIOMONITOR**

(30) Priority: 23.12.2024 US 202463737916 P
(71) Applicant: Bardy Diagnostics, Inc., Bellevue, WA 98005 (US)
(72) Inventor: PETERSON, Mercer, Seattle (US); FLOYD, Jared, Ferndale (US); CRAN, Brian, Seattle (US)
(74) Representative: Reddie & Grose LLP

(57) **Abstract**

Hydrogel containment solutions in wearable medical devices are provided. The hydrogel containment solution partially encloses a hydrogel laterally such that the hydrogel does not leave an area between an electrode and a surface of a patient's skin during use of the wearable medical device. The hydrogel containment solution may incorporate an insert to support the position of the hydrogel.

## Description

This application relates in general to medical systems, and in particular to a hydrogel retention system for wearable medical devices.

Wearable medical devices are used in a variety of applications. Such nonlimiting uses of wearable medical devices include patient monitoring, delivery of patient therapeutics, and medical diagnosis. In one example, an electrocardiogram ("ECG") monitor is worn by a patient to measure and record electrical signals emitted from the heart. Conventionally, the ECG monitor uses a standardized set format lead configuration to record the electrical signals. Electrodes often include an adhesive on one of their sides, which allows the electrodes to adhere to the skin of the patient at a desired position on the body. Also included between the electrode and the skin of the patient is a hydrogel, which improves the overall electrical conductivity of the electrode. The hydrogel may be seated in a well or receptacle, within the ECG monitor assembly, so as to stay in place during transportation and placement of the ECG monitor on a patient. While an ECG monitor is described as one example, many other wearable medical devices likewise rely on adhesive systems and hydrogels in a variety applications.

Once the wearable medical device is properly attached to the patient, the device may collect data through patient monitoring. Collected data can then be used to determine a patient's physiology through medical diagnostic procedures. For example, data collected by an ECG monitor can be used to diagnose arrhythmias or other cardiac diseases. Generally, the hydrogel provided between the patient and the electrode(s) can affect the amount and quality of data collected, which, in turn, may affect diagnostic efficacy.

However, when a wearable medical device employing a hydrogel is affixed to the patient with a particularly thin patient adhesive, the hydrogel may elute from its well or receptacle. Namely, the adhesive's thin cross-section may not create an effective wall to trap the hydrogel within its well or receptacle. Increasing the thickness of the adhesive's cross-section may result in unintended consequences, such as patient discomfort, which may cause a patient to remove the wearable medical device prematurely and degrade the overall efficacy and accuracy of the results of the wearable medical device. An increase in thickness of the adhesive's cross-section may also inadvertently increase the stiffness of the wearable medical device, causing creases to occur in the patient adhesive during transportation or wear that result in more area for the hydrogel to escape.

The present disclosure generally relates to systems and methods for the retention of hydrogels in wells of wearable medical devices.

Generally, the present disclosure solves the problem by providing different geometries and configurations for improved retention of hydrogels in wearable medical devices.

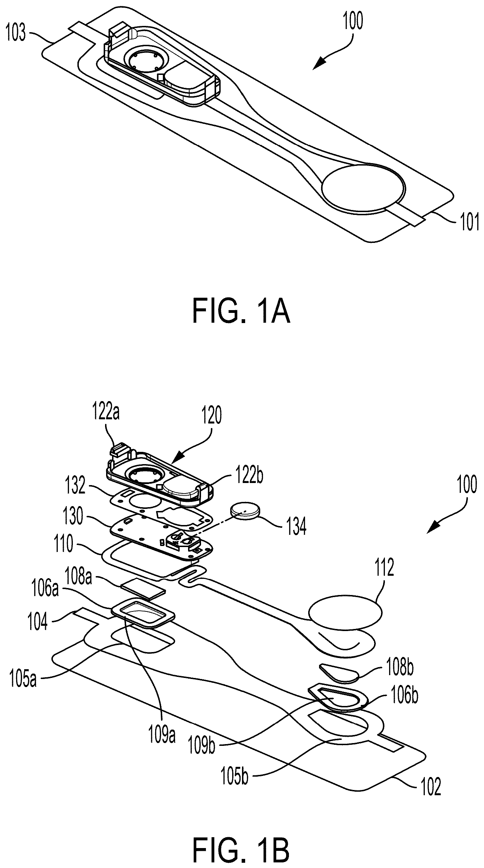

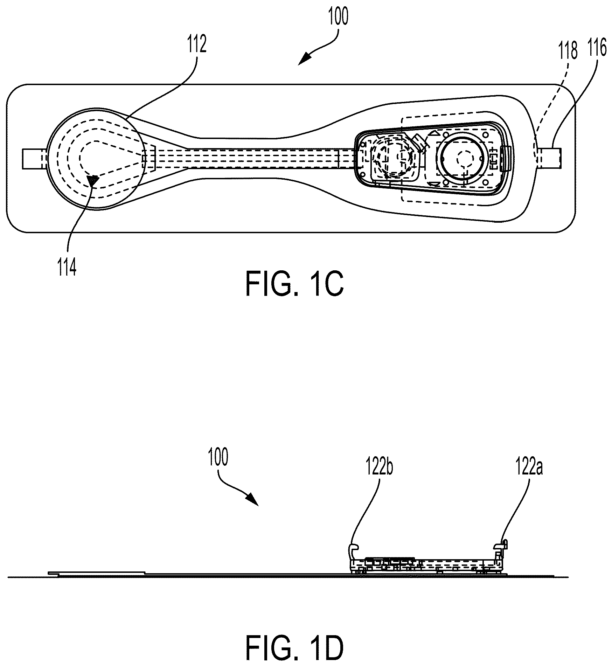

The invention will be further described by way of example with reference to the accompanying drawings, in which: FIG. 1A illustrates a perspective view of an embodiment of a biomonitor as described herein.
FIG. 1B illustrates an exploded view of the embodiment of FIG. 1A.
FIG. 1C illustrates a top view of the embodiment of FIG. 1A.
FIG. 1D illustrates a side view of the embodiment of FIG. 1A.
FIG. 1E illustrates a cross-sectional view of a distal end of the embodiment of FIG. 1A.
FIG. 1F illustrates a top view of a hydrogel and insert of a proximal end of the embodiment of FIG. 1A.
FIG. 1G illustrates a top view of a hydrogel, insert, and patient adhesive of a proximal end of the embodiment of FIG. 1A.
FIG. 1H illustrates a top view of a hydrogel and insert of a distal end of the embodiment of FIG. 1A.
FIG. 1I illustrates a top view of a hydrogel, insert, and patient adhesive of a distal end of the embodiment of FIG. 1A.
FIG. 2A illustrates a perspective view of another embodiment of a biomonitor as described herein.
FIG. 2B illustrates an exploded view of the embodiment of FIG. 2A.
FIG. 2C illustrates a top view of the embodiment of FIG. 2A.
FIG. 2D illustrates a side view of the embodiment of FIG. 2A.
FIG. 2E illustrates a cross-sectional view of a distal end of the embodiment of FIG. 2A.
FIG. 2F illustrates a top view of a hydrogel and patient adhesive of a proximal end of the embodiment of FIG. 2A.
FIG. 2G illustrates a top view of a hydrogel and patient adhesive of a distal end of the embodiment of FIG. 2A.
FIG. 3A illustrates a perspective view of another embodiment of a biomonitor as described herein.
FIG. 3B illustrates an exploded view of the embodiment of FIG. 3A.
FIG. 3C illustrates a top view of the embodiment of FIG. 3A.
FIG. 3D illustrates a side view of the embodiment of FIG. 3A.
FIG. 3E illustrates a cross-sectional view of a distal end of the embodiment of FIG. 3A.
FIG. 3F illustrates a top view of a hydrogel and hydrogel well of a proximal end of the embodiment of FIG. 3A.
FIG. 3G illustrates a top view of a hydrogel and hydrogel well of a distal end of the embodiment of FIG. 3A.
FIG. 4A illustrates a top view of an embodiment of a biomonitor as described herein.
FIG. 4B illustrates a side view of the embodiment of FIG. 4A.

As previously noted, wearable medical devices may provide patients with various benefits, including continuous monitoring of physiological signals. Data collected through continuous monitoring, can be used, for example, to detect sporadic events or facilitate diagnosis by a medical professional. However, diagnostic efficacy can be affected by the quality and amount of data collected. For example, an increase in the amount of collected data allows a physician to identify events of potential concern that may not otherwise be detected during a shorter monitoring period. In another example, a physician could more accurately determine the severity of a diagnosis by analyzing the number of occurrences over a longer time period. Therefore, by increasing the amount of data collected through long-term monitoring, diagnostic efficacy can improve. With improved diagnostic efficacy, the quality of patient care will increase.

While this described monitoring is often essential to provide a high quality of patient care, continuous monitoring over an extended period is challenging. One challenge in continuous monitoring is ensuring that the hydrogel, which facilitates electrode measurements from the chest, remains in place for the duration of patient's use. If the hydrogel is not retained, accurate data is unable to be collected. These hydrogels are often pre-loaded into the wearable medical device. These wearable medical devices often rely upon various skin-friendly adhesives in order to stay attached to a patient for the duration of monitoring or treatment, as well as to form a barrier to prevent the hydrogel from escaping. However, the interaction of the hydrogels and the adhesives can cause the adhesive to detach from the patient's skin, causing a loss of hydrogel as well as attachment problems for the system generally.

Multiple variables can affect the amount of hydrogel retained in the wearable medical device. As specified above, the interaction of the adhesive and the hydrogel may cause the adhesive to lose its effectiveness, causing the hydrogel to be lost and the wearable medical device to no longer stay on the patient's skin. The geometries and dimensions of wells or receptacles (hereinafter referred to generally as "wells" or "well") that hold the hydrogel during shipment, placement, and wear of the wearable medical device can also contribute to higher or lower hydrogel retention. Finally, the amount of adhesive used can contribute to higher or lower hydrogel retention.

The present disclosure provides a hydrogel retention system. By providing more conducive geometries, hydrogel may be retained more effectively during shipment of wearable medical devices, as well as during and after placement of the wearable medical devices on a patient. With increased hydrogel retention, the amount and quality of the data collected may increase. In turn, diagnostic efficacy may improve based on the increased quality and amount of data collected.

### Inserts

Referring to FIGS. 1A-D, a first example embodiment of the hydrogel retention system is illustrated. A biometer 100 is depicted with a distal end 101 and a proximal end 103. The biometer comprises a removable liner 102. A patient adhesive 104 comprising a proximal cutout 105a and a distal cutout 105b is removably adhered to the removable liner 102 such that during transportation the biomonitor 100 remains intact and more rigid than the patient adhesive 104 would otherwise be. The removable liner 102 also ensures that the patient adhesive 104 does not lose its adhesiveness or inadvertently stick to itself before application to a patient. The patient adhesive 104 may be any appropriate adhesive, such as, but not limited to, a textile body with an adhesive layer. The adhesive layer may be acrylic based. In some embodiments, the patient adhesive 104 may be comprised of commercially available materials. Examples of these commercially available materials may include DermaMed S-2714 - Polyurethane Nonwoven Backer with Acrylic Patient Adhesive, Lohmann DuploMED 85300 - Polyurethane Nonwoven Backer with Acrylic Patient Adhesive, or other suitable materials. The patient adhesive 104 may comprise a tab 116 configured to make it easier for an operator to remove the patient adhesive 104 from the removable liner 102. The tab 116 may be configured so as not to have an adhesive portion so that it does not adhere to the removable liner 102, making it easier for an operator to grip. Further, the patient adhesive may include perforations 118 at the base of each tab 116 such that each tab 116 may be removed from biometer 100 after application onto the patient. In this way, the biomonitor 100 may rest more comfortably when attached to a patient, without extraneous features which may tend to dispose themselves away from the patient's skin during wear and get caught on items of clothing, for example. The perforations 118 may be micro perforations.

The biomonitor 100 further comprises a circuit 110 which may be a flexible circuit. The circuit 110 has an electrode disposed at a distal end and an electrode disposed at a proximal end such that when the biomonitor 100 is adhered to a patient, the electrodes are disposed in operable position on a patient's chest. The flexible circuit 110 may include silver ink as an electrical connector and/or comprising the electrodes, and the silver ink may be disposed to include a curved portion 114. The biomonitor 100 may include a sticker 112 to ensure the distal electrode of the flexible circuit 110 does not wander on the biomonitor 100. The flexible circuit may include a serpentine portion configured to allow for stretching to occur along the length of the biomonitor 100 without disturbing the operability of the flexible circuit.

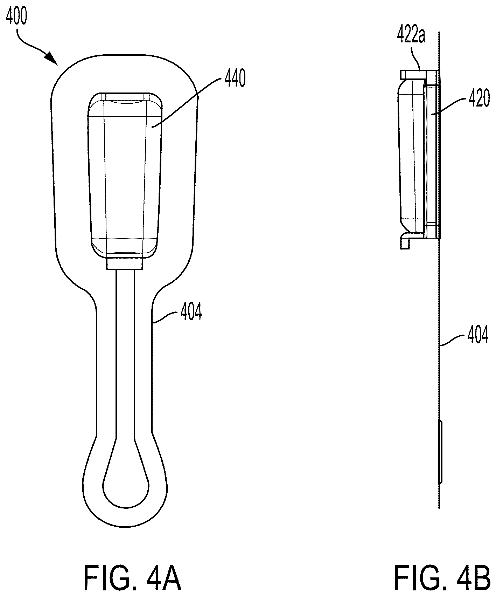

The biomonitor 100 may further include a circuit board 130, such as a printed circuit board assembly (commonly known as a "PCBA"), which includes a terminal for a battery 134. The battery 134 may provide power to the circuit board 100, and thereby to the flexible circuit 110, as well as to other electrical components of the biomonitor, such as but not limited to a processor, a memory, or an internal clock or timer. A battery seal layer 132 may also be included in the biomonitor 100. The biomonitor further comprises a housing 120 configured to accept an upper housing (such as upper housing 440 depicted in the embodiment of FIGS. 4A and B). The housing 120 may accomplish this by clips 122a, 122b disposed at a distal end and a proximal end of the housing 120. Although not pictured, a single clip 122a may be used instead, with a corresponding geometry on the housing 120 configured to keep the upper housing from shifting unintentionally. Further, more than two clips could be employed and disposed at any position on the housing 120 to achieve a similar effect. It should be appreciated that the embodiment of FIGS. 4A-B may be combined with any of the embodiments of FIGS. 1A-3E and is an illustrative example of a biomonitor 400 when assembled for patient usage. The patient adhesive 404 is removably attached to a patient when in use. The upper housing 440 may removably attach to the housing 420 by clip 422a. The upper housing 440 may further house electrical and/or computer components configured to measure and track certain health and body conditions of the patient, such as cardiac rhythm. The upper housing 440 may also further comprise a user input device configured to allow a user to mark certain events or symptoms experienced during use of the biomonitor 400.

Returning to FIGS. 1A-D, the patient adhesive 104 further comprises, in an embodiment, two cutout portions 105a, 105b disposed on a distal end and a proximal end of the patient adhesive 104. The cutout portions 105a, 105b are preferably in operable alignment with the electrodes of the flexible circuit 110. In this configuration, no patient adhesive 104 impedes the electrodes of the flexible circuit 110 from receiving a signal from a patient's skin or body when the biomonitor 100 is attached to a patient. The biomonitor 100 may further comprise inserts 106a, 106b, which may be made from acrylic, hydrocolloid, or other suitable materials, disposed at a distal end and a proximal end of the patient adhesive 104. These inserts 106a, 106b are preferably disposed in alignment with the cutout portions 105a, 105b such as to form a well for the inserts 106a, 106b to be seated inside. The inserts, in turn, have cutout portions 109a, 109b which are configured to have hydrogels 108a, 108b be seated inside. In this way, the hydrogels 108a, 108b provide an interface between the flexible circuit 110 and a patient's skin and promote efficient signal transmittance from the patient's skin to the electrodes of the flexible circuit 110. The inserts 106a, 106b seat the hydrogels 108a, 108b such that the hydrogels 108a, 108b are mechanically resilient to stresses from shipping, attachment to a patient, and wear by a patient. The inserts 106a, 106b may provide an extra surface to impede movement of the hydrogels 108a, 108b laterally as to the patient adhesive 104 and protect the hydrogels 108a, 108b from escaping the assembly between the interface of the flexible circuit 110 and the patient adhesive 104 or the interface between the patient adhesive 104 and the patient's skin. The inserts 106a, 106b may be comprised of commercially available materials. Such commercially available materials may include DermaMed S-2714 - Polyurethane Nonwoven Backer, Lohmann DuploMED 85300 - Polyurethane Nonwoven Backer, or other suitable materials.

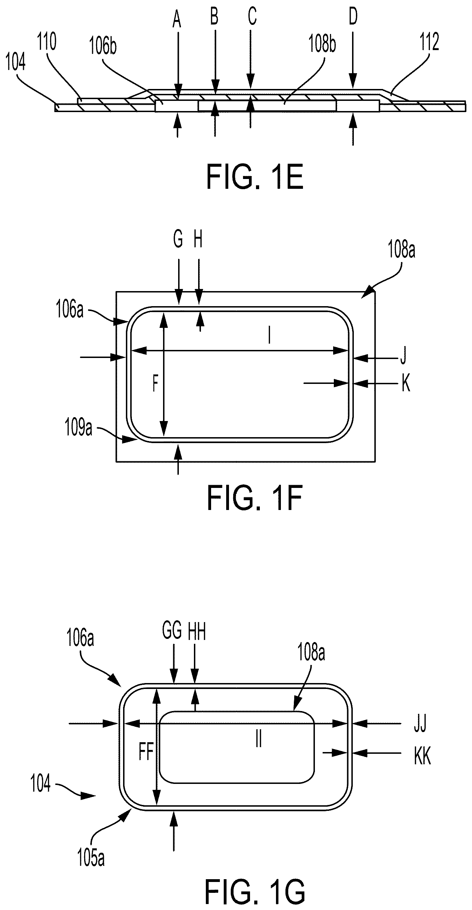

FIG. 1E illustrates a cross-section of a distal end of the biomonitor 100 in an assembled state. The insert 106b comprises a thickness A, which preferably is between 0.0268 in. and 0.0240 in., but more preferably is 0.0240 in. The flexible circuit 110 comprises a thickness B, which preferably is 0.002 in. The sticker 112 comprises a thickness C, which preferably is between 0.0102 in. and 0.0115 in., but more preferably is 0.011 in. A total thickness D comprises an addition of thicknesses A, B, and C and is between 0.015 in. and 0.075 in., but is preferably between 0.0362 in. and 0.0403 in., and more preferably is 0.0370 in.

FIG. 1F illustrates a top view of a hydrogel 108a and insert 106a of a proximal end of the biomonitor 100. The hydrogel 108a comprises a width F, which preferably is between 0.40 in. and 0.42 in., but more preferably is 0.41 in. The insert cutout 109a comprises a width G, which preferably is between 0.415 in. and 0.445 in., but more preferably is 0.430 in. The width of the insert cutout 109a and the width of hydrogel 108a create a clearance, wherein the hydrogel 108a is preferably seated at a position where the width F is centered in the width G, creating a clearance H which is preferably between -0.0225 in. and 0.0025 in. (wherein a negative clearance corresponds to an overlap), but more preferably is 0.0100 in. The hydrogel 108a also comprises a length I, which is preferably between 0.69 in. and 0.71 in., but more preferably is 0.70 in. The insert cutout 109a also comprises a length J, which is preferably between 0.705 in. and 0.735 in., but more preferably is 0.720 in. The length of the insert cutout 109a and the length of hydrogel 108a create a clearance, wherein the hydrogel 108a is preferably seated at a position where the length I is centered in the length J, creating a clearance K which is preferably between -0.0225 in. and 0.0025 in., but more preferably is 0.0100 in.

FIG. 1G illustrates a top view of a hydrogel 108a, insert 106a, and patient adhesive 104 of a proximal end of the embodiment of FIG. 1A. The insert 106a has a width FF which is preferably between 0.715 in. and 0.725 in., but more preferably is 0.720 in. The patient adhesive cutout 105a comprises a width GG, which is preferably between 0.720 in. and 0.745 in., but more preferably is 0.730 in. The width of the patient adhesive cutout 105a and the width of insert 106a create a clearance, wherein the insert 106a is preferably seated at a position where the width FF is centered in the width GG, creating a clearance HH which is preferably between -0.0225 in. and 0.015 in., but more preferably is 0.0050 in. The insert 106a also comprises a length II which is preferably between 1.005 in. and 1.015 in., but more preferably is 1.01 in. The patient adhesive cutout 105a comprises a length JJ which is preferably between 1.010 in. and 1.035 in., but more preferably is 1.020 in. The length of the patient adhesive cutout 105a and the length of insert 106a create a clearance, wherein the insert 106a is preferably seated at a position where the length II is centered in the length JJ, creating a clearance KK which is preferably between -0.0225 in. and 0.0150 in., but more preferably is 0.0050 in.

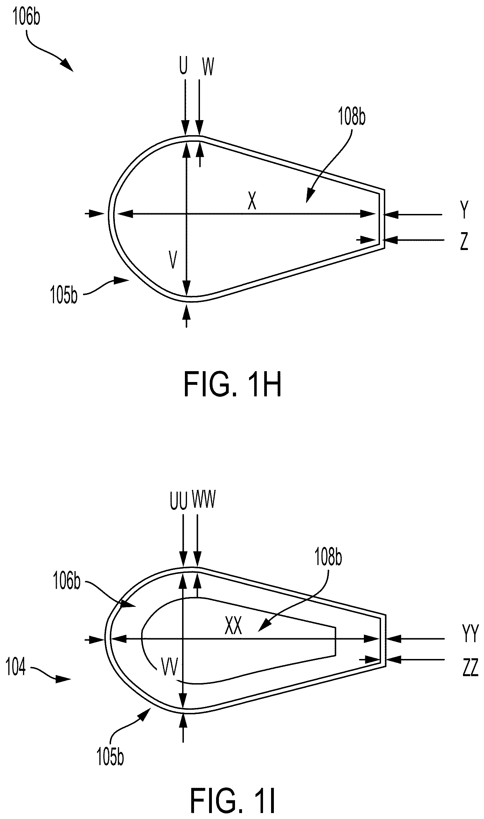

FIG. 1H illustrates a top view of a hydrogel 108b and insert 106b of a distal end of the embodiment of FIG. 1A. The hydrogel 108b comprises a width V which is preferably between 0.455 in. and 0.485 in., but more preferably is 0.470 in. The insert cutout 109b comprises a width U, which preferably is between 0.44 in. and 0.46 in., but more preferably is 0.45 in. The width of the insert cutout 109b and the width of hydrogel 108b create a clearance, wherein the hydrogel 108b is preferably seated at a position where the width V is centered in the width U, creating a clearance W which is preferably between -0.0225 in. and 0.0025 in., but more preferably is 0.0100 in. The hydrogel 108b comprises a length X which is preferably between 0.79 in. and 0.81 in., but more preferably is 0.80 in. The insert cutout 109b comprises a length Y which is preferably between 0.805 in. and 0.835 in., but more preferably is 0.820 in. The length of the insert cutout 109b and the length of hydrogel 108b create a clearance, wherein the hydrogel 108b is preferably seated at a position where the length X is centered in the length Y, creating a clearance Z which is preferably between - 0.0225 in. and 0.0025 in., but more preferably is 0.0100 in.

FIG. 1I illustrates a top view of a hydrogel 108b, insert 106b, and patient adhesive 104 of a distal end of the embodiment of FIG. 1A. The insert 106b has a width VV which is preferably between 0.755 in. and 0.765 in., but more preferably is 0.760 in. The patient adhesive cutout 105b comprises a width UU, which is preferably between 0.760 in. and 0.785 in., but more preferably is 0.770 in. The width of the patient adhesive cutout 105b and the width of insert 106b create a clearance, wherein the insert 106b is preferably seated at a position where the width VV is centered in the width UU, creating a clearance WW which is preferably between -0.0225 in. and 0.015 in., but more preferably is 0.0050 in. The insert 106b also comprises a length XX which is preferably between 1.105 in. and 1.115 in., but more preferably is 1.11 in. The patient adhesive cutout 105b comprises a length YY which is preferably between 1.110 in. and 1.135 in., but more preferably is 1.120 in. The length of the patient adhesive cutout 105b and the length of insert 106b create a clearance, wherein the insert 106a is preferably seated at a position where the length XX is centered in the length YY, creating a clearance ZZ which is preferably between -0.0225 in. and 0.0150 in., but more preferably is 0.0050 in.

### Overlapping Hydrogels

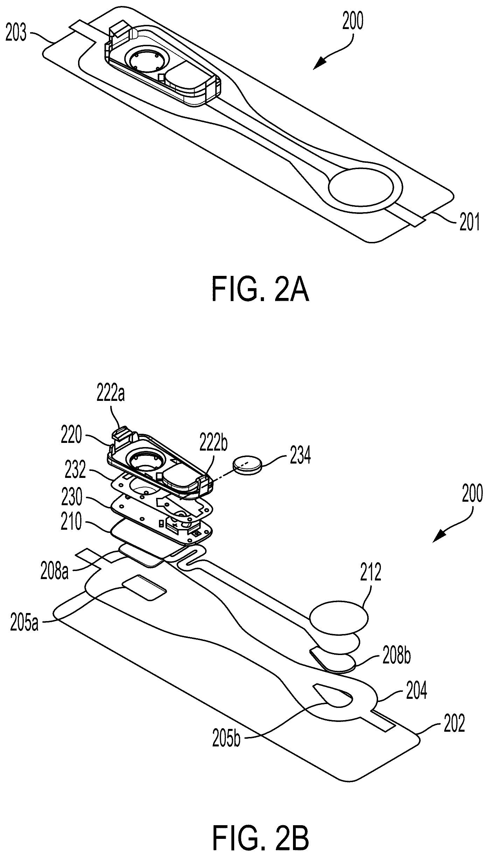

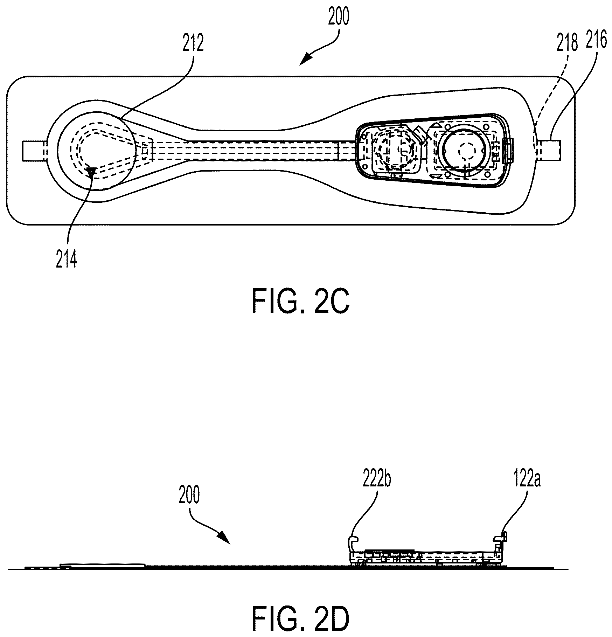

Turning now to FIGS. 2A-D, a different embodiment of a biomonitor 200 with a different system for the retention of hydrogels 208a, 208b is depicted. The biomonitor comprises a distal end 201 and proximal end 203. The biomonitor 200 further comprises a removable liner 202, a circuit board 230, battery 234, battery seal 232, housing 220 (comprising clips 222a, 222b), flexible circuit 210 comprising silver ink with a curved portion 214, and sticker 212 in a similar arrangement to that of the embodiment of FIGS. 1A-D.

The biomonitor 200 further comprises a patient adhesive 204, which comprises two cutout portions 205a, 205b, disposed on a distal end and a proximal end of the patient adhesive 204 and preferably in operable alignment with the electrodes of the flexible circuit 210 such that no patient adhesive 204 impedes the electrodes of the flexible circuit 210 from receiving a signal from a patient's skin or body when the biomonitor 200 is attached to a patient. The patient adhesive 204 may further comprise tab 216 to aid an operator in removing the removable liner 202 from the biomonitor 200. The patient adhesive 204 may have perforations 218 at the base of the tab 216, similar to those of the embodiment of FIGS. 1A-1D. The patient adhesive 204 may be acrylic-based.

However, this embodiment does not include inserts to aid in the retention of hydrogels 208a, 208b. Rather, the hydrogels 208a, 208b are configured with dimensions that have an area that exceed an area of the cutout portions 205a, 205b to which they correspond. For example, the hydrogels 208a, 208b may be sized to that an overlapping lip is formed around a perimeter of each hydrogel 208a, 208b and each corresponding cutout portion 205a, 205b. This provides mechanical resilience against lateral movement of the hydrogels 208a, 208b when the biomonitor 200 is assembled, such that it is less likely for the hydrogels 208a, 208b to escape between an interface of the patient adhesive 204 and a patient's skin while the biomonitor is attached to a patient, or to escape between an interface of the patient adhesive 204 and the removable liner 202 during transportation. In a preferred embodiment, the hydrogels 208a, 208b are configured so that their entire perimeters form an overlapping lip with the entire perimeters of the cutout portions 205a, 205b. It is also contemplated that the hydrogels 208a, 208b are configured so that only a portion of their perimeters form an overlapping lip with a portion of the perimeters of the cutout portions 205a, 205b. The geometries of the hydrogels 208a, 208b may also form a tab that forms an overlapping region with a portion of the perimeters of the cutout portions 205a, 205b. Further, a plurality of tabs may be employed in any configuration (such as at equidistant degrees about a center of area of the hydrogels 208a, 208b).

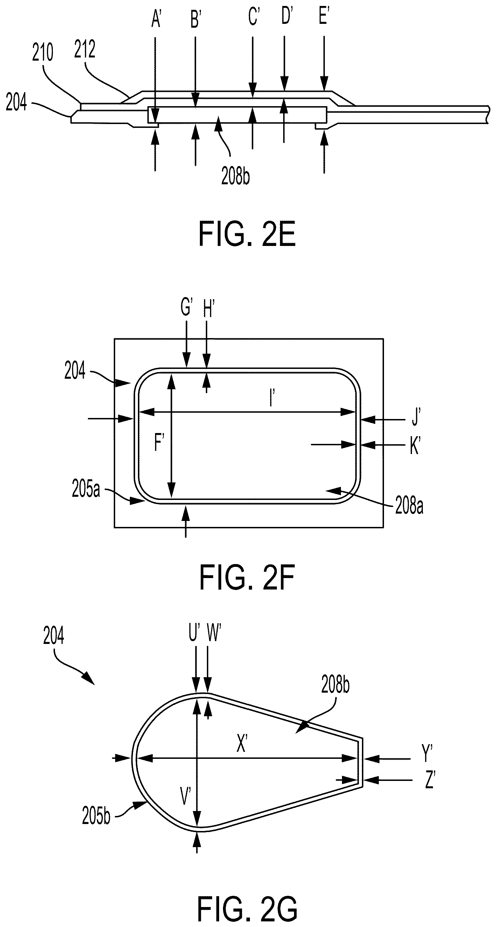

FIG. 2E illustrates a cross-section of a distal end of the biomonitor 200 in an assembled state. A region where the hydrogel 208b overlaps the patient adhesive 204 comprises a thickness A', which preferably is between 0.0102 in. and 0.0115 in., but more preferably is 0.011 in. The hydrogel 208b comprises a thickness B', which preferably is between 0.035 in. and 0.040 in., but more preferably is 0.040 in. The flex circuit 210 comprises a thickness C', which preferably is 0.002 in. The sticker 212 comprises a thickness D', which preferably is between 0.0102 in. and 0.0115 in., but more preferably is 0.0110 in. A total thickness E' comprises an addition of thicknesses A', B', C' and D' and is between 0.015 in. and 0.075 in., but is preferably between 0.0574 in. and 0.0650 in., and more preferably is 0.0640 in.

FIG. 2F illustrates a top view of a hydrogel 208a and patient adhesive 204 of a proximal end of the biomonitor 200. The hydrogel 208a comprises a width F', which preferably is between 0.50 in. and 0.52 in., but more preferably is 0.51 in. The patient adhesive cutout 205a comprises a width G', which preferably is between 0.420 in. and 0.445 in., but more preferably is 0.430 in. The width of the patient adhesive cutout 205a and the width of hydrogel 208a create a clearance, wherein the hydrogel 208a is preferably seated at a position where the width F' is centered in the width G', creating a clearance H' which is preferably between -0.050 in. and -0.0275 in. (wherein a negative clearance corresponds to an overlap), but more preferably is -0.0400 in. The hydrogel 208a also comprises a length I', which is preferably between 0.79 in. and 0.81 in., but more preferably is 0.80 in. The patient adhesive cutout 205a also comprises a length J', which is preferably between 0.710 in. and 0.735 in., but more preferably is 0.720 in. The length of the patient adhesive cutout 205a and the length of hydrogel 208a create a clearance, wherein the hydrogel 208a is preferably seated at a position where the length I' is centered in the length J', creating a clearance K' which is preferably between -0.050 in. and -0.0275 in., but more preferably is -0.0400 in.

FIG. 2G illustrates a top view of a hydrogel 208b and patient adhesive 204 of a distal end of the biomonitor 200. The hydrogel 208b comprises a width V' which is preferably between 0.460 in. and 0.485 in., but more preferably is 0.470 in. The patient adhesive cutout 205b comprises a width U', which preferably is between 0.54 in. and 0.56 in., but more preferably is 0.55 in. The width of the patient adhesive cutout 205b and the width of hydrogel 208b create a clearance, wherein the hydrogel 208b is preferably seated at a position where the width V' is centered in the width U', creating a clearance W' which is preferably between -0.050 in. and -0.0275 in., but more preferably is -0.0400 in. The hydrogel 208b comprises a length X' which is preferably between 0.89 in. and 0.91 in., but more preferably is 0.90 in. The patient adhesive cutout 205b comprises a length Y' which is preferably between 0.810 in. and 0.835 in., but more preferably is 0.820 in. The length of the patient adhesive cutout 205b and the length of hydrogel 208b create a clearance, wherein the hydrogel 208b is preferably seated at a position where the length X' is centered in the length Y', creating a clearance Z' which is preferably between -0.050 in. and -0.0275 in., but more preferably is - 0.0400 in.

### Hydrogel Wells

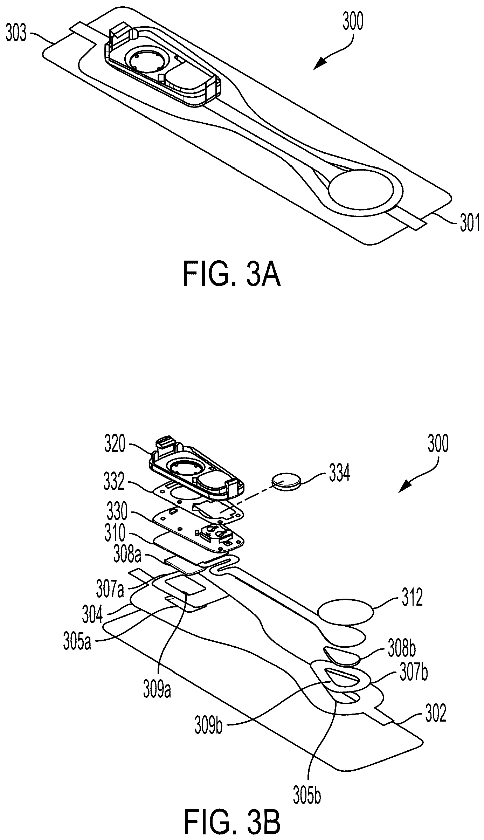

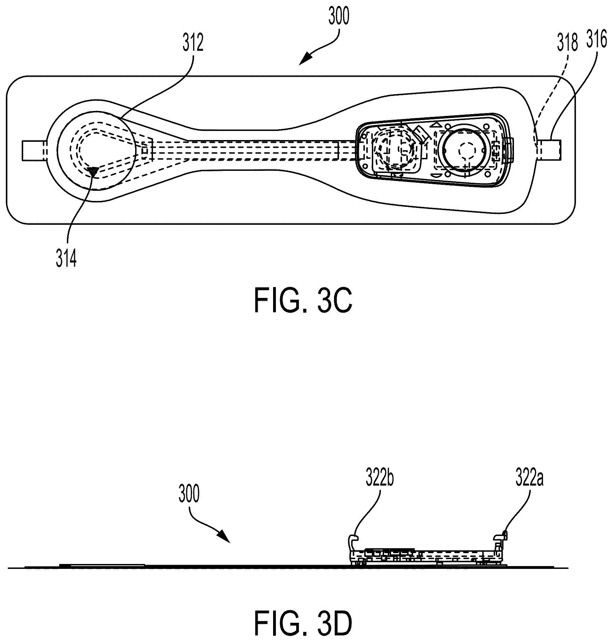

Turning now to FIGS. 3A-D, a different embodiment of a biomonitor 300 with a different system for the retention of hydrogels 308a, 308b is depicted. The biomonitor comprises a distal end 301 and proximal end 303. The biomonitor 300 further comprises a removable liner 302, a circuit board 330, battery 334, battery seal 332, housing 320 (comprising clips 322a, 322b), flexible circuit 310 comprising silver ink with a curved portion 314, and sticker 312 in a similar arrangement to that of the embodiment of FIGS. 1A-D.

The biomonitor 300 further comprises a patient adhesive 304, which comprises two cutout portions 305a, 305b, disposed on a distal end and a proximal end of the patient adhesive 304 and preferably in operable alignment with the electrodes of the flexible circuit 310 such that no patient adhesive 304 impedes the electrodes of the flexible circuit 310 from receiving a signal from a patient's skin or body when the biomonitor 300 is attached to a patient. The patient adhesive 304 may further comprise tab 316 to aid an operator in removing the removable liner 302 from the biomonitor 300. The patient adhesive 304 may have perforations 318 at the base of the tab 316, similar to those of the embodiment of FIGS. 1A-1D.

The biomonitor 300 further comprises hydrogel wells 307a, 307b, which may further comprise cutout portions 309a, 309b, corresponding to the patient adhesive cutout portions 305a, 305b. The hydrogel wells 307a, 307b seat the hydrogels 308a, 308b inside the hydrogel well cutouts 309a, 309b, such that the hydrogels 308a, 308b are mechanically resilient to stresses from shipping, attachment to a patient, and wear by a patient. The hydrogel wells 307a, 307b may provide an extra surface to impede movement of the hydrogels 308a, 308b laterally as to the patient adhesive 304 and protect the hydrogels 308a, 308b from escaping the assembly between the interface of the flexible circuit 310 and the patient adhesive 304 or the interface between the patient adhesive 304 and the patient's skin. The hydrogel wells 307a, 307b may comprise the same material as the patient adhesive 304, effectively forming an area of double-thickness patient adhesive 304 surrounding the hydrogels 308a, 308b. The hydrogel wells 307a, 307b and the patient adhesive 304 may comprise a combination of nonwoven backer and acrylic adhesive. For example, commercially available materials for the hydrogel wells 307a, 307b may include DermaMed S-2714 - Polyurethane Nonwoven Backer with Acrylic Patient Adhesive, Lohmann DuploMED 85300 - Polyurethane Nonwoven Backer with Acrylic Patient Adhesive, or other suitable materials. In this way, the hydrogel wells 307a, 307b aid the hydrogels 308a, 308b in being mechanically resilient to stresses from shipping, attachment to a patient, and wear by a patient. The hydrogel wells 307a, 307b may provide an extra surface to impede movement of the hydrogels 308a, 308b laterally as to the patient adhesive 304 and protect the hydrogels 308a, 308b from escaping the assembly between the interface of the flexible circuit 310 and the patient adhesive 304 or the interface between the patient adhesive 304 and the patient's skin.

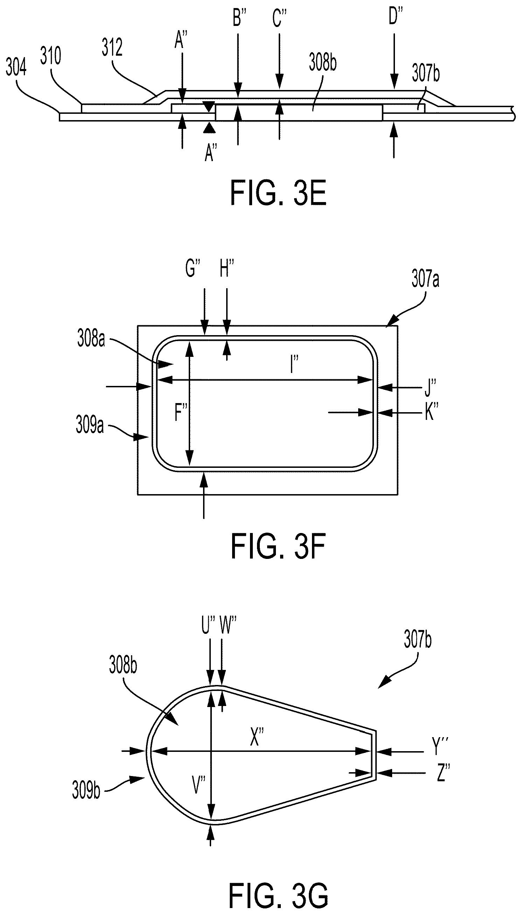

FIG. 3E illustrates a cross-section of a distal end of the biomonitor 300 in an assembled state. The patient adhesive 304 comprises a thickness A", which preferably is between 0.0101 in. and 0.0105 in., but more preferably is 0.0102 in. or 0.0104 in. In the embodiment of FIG. 3E, the hydrogel well 307b comprises the same material as the patient adhesive 304. Thus, the hydrogel well 307b also comprises a thickness A". The flexible circuit 310 comprises a thickness B", which preferably is 0.002 in. The sticker 312 comprises a thickness C", which preferably is between 0.0102 in. and 0.0115 in., but more preferably is 0.011 in. A total thickness D" comprises an addition of thicknesses B", C", and (in the embodiment of FIG. 3E) two instances of A", and is between 0.015 in. and 0.075 in., but preferably between 0.0362 in. and 0.0365 in., and more preferably is 0.0350 in.

FIG. 3F illustrates a top view of a hydrogel 308a and hydrogel well 307a of a proximal end of the biomonitor 300. The hydrogel 308a comprises a width F", which preferably is between 0.40 in. and 0.42 in., but more preferably is 0.41 in. The hydrogel well cutout 309a comprises a width G", which preferably is between 0.420 in. and 0.445 in., but more preferably is 0.430 in. The width of the hydrogel well cutout 309a and the width of hydrogel 308a create a clearance, wherein the hydrogel 308a is preferably seated at a position where the width F" is centered in the width G", creating a clearance H" which is preferably between 0.0000 in. and 0.0225 in., but more preferably is 0.0100 in. The hydrogel 308a also comprises a length I", which is preferably between 0.69 in. and 0.71 in., but more preferably is 0.70 in. The hydrogel well cutout 309a also comprises a length J", which is preferably between 0.710 in. and 0.735 in., but more preferably is 0.720 in. The length of the hydrogel well cutout 309a and the length of hydrogel 308a create a clearance, wherein the hydrogel 308a is preferably seated at a position where the length I" is centered in the length J", creating a clearance K" which is preferably between 0.0000 in. and 0.0225 in., but more preferably is 0.0100 in.

FIG. 3G illustrates a top view of a hydrogel 308b and hydrogel well 307b of a distal end of the biomonitor 300. The hydrogel 308b comprises a width V" which is preferably between 0.460 in. and 0.485 in., but more preferably is 0.470 in. The hydrogel well cutout 309b comprises a width U", which preferably is between 0.44 in. and 0.46 in., but more preferably is 0.45 in. The width of the hydrogel well cutout 309b and the width of hydrogel 308b create a clearance, wherein the hydrogel 308b is preferably seated at a position where the width V" is centered in the width U", creating a clearance W" which is preferably between 0.0000 in. and 0.0225 in., but more preferably is 0.0100 in. The hydrogel 308b comprises a length X" which is preferably between 0.79 in. and 0.81 in., but more preferably is 0.80 in. The hydrogel well cutout 309b comprises a length Y" which is preferably between 0.810 in. and 0.835 in., but more preferably is 0.820 in. The length of the hydrogel well cutout 309b and the length of hydrogel 308b create a clearance, wherein the hydrogel 308b is preferably seated at a position where the length X" is centered in the length Y", creating a clearance Z" which is preferably 0.0000 in. and 0.0225 in., but more preferably is 0.0100 in.

An aspect of the present disclosure provides a biomonitor for measuring one or more physiological signals. For example, the one or more physiological signals may include, but are not limited to, heart rate, oxygen saturation, airflow, respiratory effort, temperature, motion detection, blood pressure, and combinations thereof. The one or more physiological signals may be used to determine specific disease states. For example, the biomonitor may be used to detect one or more of atrial fibrillation, sleep apnea, fainting, falling, heart failure, chronic obstructive pulmonary disease.

In some examples, the biomonitor is an atrial fibrillation and/or sleep apnea detector. Obstructive Sleep Apnea ("OSA") is highly associated with atrial fibrillation. Individuals with OSA are 4 times more likely to have atrial fibrillation. OSA cannot be diagnosed merely by measuring pulse oximetry but also requires measurement of airflow and respiratory effort. The atrial fibrillation and/or sleep apnea monitor may include an ECG sensor, a pulse oximetry sensor, and an acoustic sensor, such as a microphone.

In some examples, the biomonitor is a heart failure and deterioration detector. Diagnosing heart failure or monitoring deterioration of the heart can reduce expensive hospitalizations. The heart failure and deterioration detector may include an ECG sensor, an acoustic sensor to act as a digital stethoscope, a motion detector, a blood pressure sensor, and a respiratory rate sensor.

In some examples, the biomonitor is a fall detector, such as to detect when a user faints. The fall detector may include a motion sensor, an ECG sensor, a pulse oximetry sensor, and a blood pressure sensor.

In some examples, the biomonitor is a Chronic Obstructive Pulmonary Disease ("COPD") detector. COPD is the third leading cause of death worldwide and diagnosis and monitoring techniques for COPD are expensive and time consuming. The COPD detector may include multi-modal sensing capability, such as a digital stethoscope, an ECG sensor, a thermometer, and a motion detector.

Certain numerical ranges are presented in the embodiments. Unless stated otherwise, these numerical ranges are inclusive of the minimums and maximums described.

Embodiments of the invention can be described with reference to the following numbered clauses, with preferred features laid out in in the dependent clauses:
Clause 1: A wearable medical device comprising:
   an electrical circuit with an electrode;
   a patient adhesive;
   a hydrogel; and
   a hydrogel well,
   wherein the patient adhesive is configured to removably affix the wearable medical device to a patient,
   wherein the hydrogel well defines a well configured to partially enclose the hydrogel laterally while the wearable medical device is removably affixed to the patient, and wherein the patient adhesive is configured to be disposed between the hydrogel well and a skin surface of the patient while the wearable medical device is removably affixed to the patient,
   wherein the patient adhesive defines a well configured to partially enclose the hydrogel laterally while the wearable medical device is removably affixed to the patient, and
   wherein the hydrogel is configured to be disposed between the electrode and the skin surface of the patient while the wearable medical device is removably affixed to the patient.
Clause 2: The wearable medical device of Clause 1, further comprising a sticker, wherein the sticker is disposed toward a distal end of the wearable medical device at least partially on a surface of the electrode which is furthest from the skin surface of the patient while the wearable medical device is removably affixed to the patient.
Clause 3: The wearable medical device of Clause 1, further comprising a removable liner configured to be removably affixed to the patient adhesive,
   wherein a thickness between a surface of the removable liner removably affixed to the patient adhesive and a surface of the wearable medical device furthest from the removable liner on a distal side is between 0.015 in. and 0.075 in.
Clause 4: The wearable medical device of Clause 3, wherein the thickness is 0.0350 in.
Clause 5: The wearable medical device of Clause 1, wherein the electrical circuit is a flexible circuit comprising a portion with a serpentine geometry.
Clause 6: The wearable medical device of Clause 1, wherein the wearable medical device is a biomonitor.
Clause 7: The wearable medical device of Clause 1, wherein the patient adhesive comprises a material, wherein the hydrogel well comprises a material, and wherein the patient adhesive material and the hydrogel well material comprise the same material.
Clause 8: A wearable medical device comprising:
   an electrical circuit with an electrode;
   a patient adhesive;
   a hydrogel; and
   an insert,
   wherein the patient adhesive is configured to removably affix the wearable medical device to a patient,
   wherein the insert defines a well configured to enclose the hydrogel laterally while the wearable medical device is removably affixed to the patient,
   wherein the patient adhesive defines a second well configured to enclose the insert laterally while the wearable medical device is removably affixed to the patient,
   wherein the hydrogel is configured to be disposed between the electrode and a skin surface of the patient while the wearable medical device is removably affixed to the patient.
Clause 9: The wearable medical device of Clause 8, further comprising a sticker, wherein the sticker is disposed toward a distal end of the wearable medical device at least partially on a surface of the electrode which is furthest from the skin surface of the patient while the wearable medical device is removably affixed to the patient.
Clause 10: The wearable medical device of Clause 8, further comprising a removable liner configured to be removably affixed to the patient adhesive,
   wherein a thickness between a surface of the removable liner removably affixed to the patient adhesive and a surface of the wearable medical device furthest from the removable liner on a distal side is between 0.015 in. and 0.075 in.
Clause 11: The wearable medical device of Clause 10, wherein the thickness is 0.0370 in.
Clause 12: The wearable medical device of Clause 8, wherein the electrical circuit is a flexible circuit comprising a portion with a serpentine geometry.
Clause 13: The wearable medical device of Clause 8, wherein the wearable medical device is a biomonitor.
Clause 14: A wearable medical device comprising:
   an electrical circuit with an electrode;
   a patient adhesive; and
   a hydrogel,
   wherein the patient adhesive is configured to removably affix the wearable medical device to a patient,
   wherein the patient adhesive defines a well, and wherein the hydrogel defines a perimeter larger than a perimeter of the well of the patient adhesive, such that a portion of the patient adhesive is disposed between the hydrogel and a skin surface of the patient while the wearable medical device is removably affixed to the patient,
   wherein the hydrogel is configured to be partially disposed between the electrode and the skin surface of the patient while the wearable medical device is removably affixed to the patient.
Clause 15: The wearable medical device of Clause 14, further comprising a sticker, wherein the sticker is disposed toward a distal end of the wearable medical device at least partially on a surface of the electrode which is furthest from the skin surface of the patient while the wearable medical device is removably affixed to the patient.
Clause 16: The wearable medical device of Clause 14, further comprising a removable liner configured to be removably affixed to the patient adhesive,
   wherein a thickness between a surface of the removable liner removably affixed to the patient adhesive and a surface of the wearable medical device furthest from the removable liner on a distal side is between 0.015 in. and 0.075 in.
Clause 17: The wearable medical device of Clause 16, wherein the thickness is 0.0640 in.
Clause18: The wearable medical device of Clause 14, wherein the electrical circuit is a flexible circuit comprising a portion with a serpentine geometry.
Clause 19: The wearable medical device of Clause 14, wherein the wearable medical device is a biomonitor.
Clause 20: The wearable medical device of Clause 14, wherein the entire perimeter of the well of the patient adhesive is disposed between the hydrogel and a skin surface of the patient while the wearable medical device is removably affixed to the patient.

## Claims

1. A wearable medical device comprising:
an electrical circuit with an electrode;
a patient adhesive;
a hydrogel; and
wherein the patient adhesive is configured to removably affix the wearable medical device to a patient,
wherein the well is configured to partially enclose the hydrogel laterally while the wearable medical device is removably affixed to the patient,
and wherein the hydrogel is configured to be disposed between the electrode and a skin surface of the patient while the wearable medical device is removably affixed to the patient.

2. The wearable medical device of Claim 1, wherein the patient adhesive defines a second well configured to enclose the insert laterally while the wearable medical device is removably affixed to the patient.

3. The wearable medical device of claim 1 wherein the well is a hydrogel well,
wherein the patient adhesive is configured to removably affix the wearable medical device to a patient,
and wherein the patient adhesive defines a well configured to partially enclose the hydrogel laterally while the wearable medical device is removably affixed to the patient.

4. The wearable medical device of Claim 3, wherein the patient adhesive comprises a material, wherein the hydrogel well comprises a material, and wherein the patient adhesive material and the hydrogel well material comprise the same material.

5. The wearable medical device of claim 1
wherein the patient adhesive defines a well, and wherein the hydrogel defines a perimeter larger than a perimeter of the well of the patient adhesive, such that a portion of the patient adhesive is disposed between the hydrogel and a skin surface of the patient while the wearable medical device is removably affixed to the patient,

6. The wearable medical device of Claim 5, wherein the entire perimeter of the well of the patient adhesive is disposed between the hydrogel and a skin surface of the patient while the wearable medical device is removably affixed to the patient.

7. The wearable medical device of any preceding claim, further comprising a sticker, wherein the sticker is disposed toward a distal end of the wearable medical device at least partially on a surface of the electrode which is furthest from the skin surface of the patient while the wearable medical device is removably affixed to the patient.

8. The wearable medical device of any preceding claim, further comprising a removable liner configured to be removably affixed to the patient adhesive,
wherein a thickness between a surface of the removable liner removably affixed to the patient adhesive and a surface of the wearable medical device furthest from the removable liner on a distal side is between 0.015 in. and 0.075 in.

9. The wearable medical device of any preceding claim, wherein the thickness is 0.0350 in.

10. The wearable medical device of any preceding claim, wherein the electrical circuit is a flexible circuit comprising a portion with a serpentine geometry.

11. The wearable medical device of any preceding claim, wherein the wearable medical device is a biomonitor.
